# EUROPEAN PATENT SPECIFICATION

(11) **EP 1 571 935 B1**
(45) Date of publication and mention of the grant of the patent: **28.03.2012**
(21) Application number: 03776303.4
(22) Date of filing: 09.10.2003
(51) Int. Cl.: A41B 9/00, A41B 9/04, A61F 13/15

(54) **LINER FOR USE IN COMBINATION WITH THONG, G-STRING, TANGA OR T-BACK UNDERWEAR**
EINLAGE IN KOMBINATION MIT TANGASLIP
PROTEGE-SLIP DESTINE A ETRE UTILISE EN ASSOCIATION AVEC UN MINI-SLIP, UN STRING, UN TANGA OU UN SOUS-VETEMENT DE TYPE CACHE-PUDEUR

(30) Priority: 27.11.2002 US 306045
(43) Date of publication of application: 14.09.2005
(73) Proprietor: KIMBERLY-CLARK WORLDWIDE, INC., Neenah, WI 54956 (US)
(72) Inventor: BELL, Daryl, S., Appleton, WI 54915 (US); CASSON, Kenneth, R., Menasha, WI 54952 (US); LEMERE, Jeremy, S., DePere, WI 54115 (US)
(74) Representative: Mabey, Katherine Frances
(86) International application number: PCT/US2003/032226
(87) International publication number: WO 2004/049838

(56) References cited:
- WO-A-01/49232
- WO-A-98/51249
- WO-A-03/022082
- US-A- 5 114 419
- US-A- 5 467 482
- US-A- 5 683 373

## Description

### BACKGROUND OF THE INVENTION

This invention generally relates to feminine hygiene products, and more particularly to a liner sized and shaped for insertion into a particular type of women's underwear known as either a thong, a g-string, a tanga or a T-back (hereinafter "thong underwear"). Such underwear differs from traditionally shaped women's underwear (e.g., briefs, bikinis) in that it includes only a thin web of material passing between the buttocks, rather than a wider piece of material extending laterally outward in the rear to cover the buttocks. Thong underwear also generally includes a narrower crotch portion than traditionally shaped underwear. Thong underwear is popular with consumers because the thin web of material extending between the buttocks and the narrower crotch portion is less likely to reveal a panty line (i.e., an edge seam of the underwear) through tight-fitting outer garments, thereby providing a smoother appearance for garments worn over the thong underwear.

Conventional pantyliners and menstrual pads, generally liners, are designed for traditionally shaped underwear. For example, conventional liners are hourglass-shaped to generally match the hourglass shape of traditional underwear. When worn, a narrower middle portion of the conventional liner and underwear fit between the legs of the wearer while the wider end portions extend forward and rearward from between the legs. When inserted into traditionally shaped underwear, such hourglass-shaped liners are fully contained within the confines of the underwear. In other words, lateral edges of the liner do not extend beyond the lateral edges of the underwear. This is an important characteristic, because portions of the liner extending beyond the edges of the underwear can irritate the wearer's skin. When inserted into thong underwear, however, such hourglass-shaped liners cause several problems. First, a conventional liner is generally too large for full insertion into the crotch portion without extending laterally beyond the edge of the underwear. Second, the rearward extending portion of the liner extends onto the thin web of the thong underwear, forcing a large portion of the liner to extend beyond the edge of the web. This excess material can irritate the wearer by rubbing and chaffing the wearer's legs.

Previous attempts to provide a liner suitable for thong underwear have suffered from various drawbacks. Many such liners are wider than the crotch portion of the thong underwear, typically near a lower end of the crotch portion. These designs cause an excess of liner material to extend beyond the edge of the thong underwear. As noted above, such excess material can irritate the wearer. Other liners do not widen sufficiently near an upper end of the liner to provide adequate coverage sufficient to absorb all bodily fluids. Still other liners extend onto the web of the thong underwear, which may be uncomfortable when placed between the buttocks of the wearer. Again, because such liners extend laterally beyond the edges of the thong underwear, they cause rubbing and chaffing that may irritate the wearer.

WO01/49232 discloses an absorbent product with fastening arrangements. US 5,683,373 discloses a sanitary napkin shaped for use with a thong garment.

The present invention provides a liner in accordance with claim 1.

In general, the invention may provide a liner in combination with a garment (e.g., thong underwear) for fitting about a wearer's waist and crotch. The garment includes a waistband for encircling the waist of the wearer and a crotch portion which extends downward from the waistband for positioning over the crotch of the wearer. The crotch portion includes a left garment edge and a right garment edge converging downward from the waistband toward a lower end of the crotch portion. A web extends rearward from the lower end of the crotch portion for passing between the buttocks of the wearer and attaching to the waistband. The liner is adapted for insertion into the crotch portion for absorbing bodily fluids and protecting the garment from such fluids. The liner is generally flat and includes a garment-facing surface adapted for flatwise contact with the crotch portion of the garment and a body-facing surface adapted for flatwise contact with the crotch of the wearer. The liner further includes a left liner edge and a right liner edge converging downward toward an apex of the liner. The liner has a size and configuration such that upon insertion of the liner into the crotch portion of the garment, the liner apex contacts the crotch portion at a location adjacent the lower end of the crotch portion, the left liner edge does not extend laterally out beyond the left garment edge, the right liner edge does not extend laterally out beyond the right garment edge, and the liner apex is not overlapping with the web.

In embodiments of the invention, each of the left and right edges of the liner has a substantially convex portion.

In embodiments of the invention, the liner has an apex, an arcuate upper liner edge and an angle of inclusion between the left liner edge and the right liner edge is between about 27° and about 35°.

Other objects and features will be in part apparent and in part pointed out hereinafter.

### BRIEF DESCRIPTION OF THE DRAWINGS

Fig. 1 is a front elevation of a prior art liner inserted into thong underwear;
Fig. 2 is a perspective of a liner not in accordance with the present invention upon insertion into thong underwear;
Fig. 3 is a perspective of a wearer wearing the liner and thong underwear of Fig. 2;
Fig. 4 is a partial plan view of the thong underwear and liner of Fig. 2 in a flattened condition;
Fig. 5 is a partial plan view of the thong underwear and another liner in accordance with the present invention in a flattened condition; and
Figs. 6, 8 and 9 are plan views of various liners of the present invention; and
Figure 7 is a plan view of a liner not in accordance with the invention.

Corresponding reference characters indicate corresponding parts throughout the several views of the drawings.

### DETAILED DESCRIPTION OF THE PREFERRED EMBODIMENTS

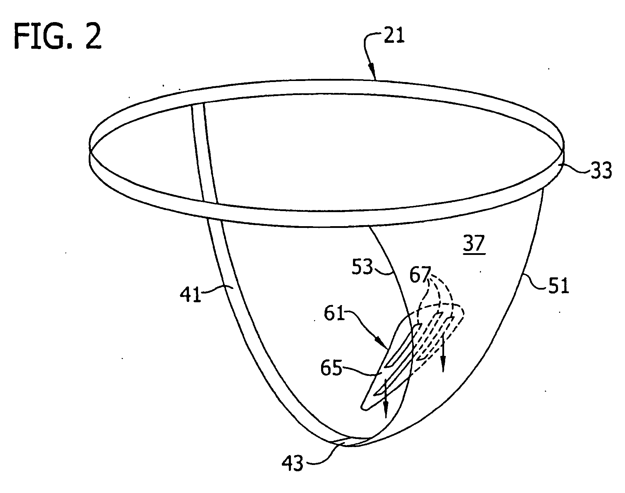

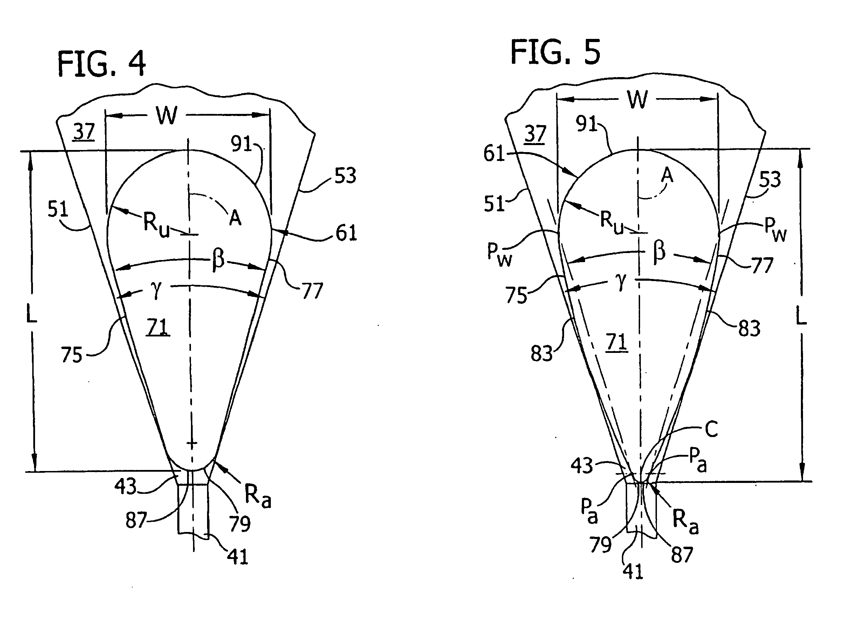

In accordance with the invention the left and right liner edges both include a substantially convex portions. Figures 5, 6, 8 and 9 illustrate liners in accordance with the invention. Figures 2 to 4 and 7 illustrate liners not in accordance with the invention, but which have been retained to facilitate understanding of certain features of the invention.

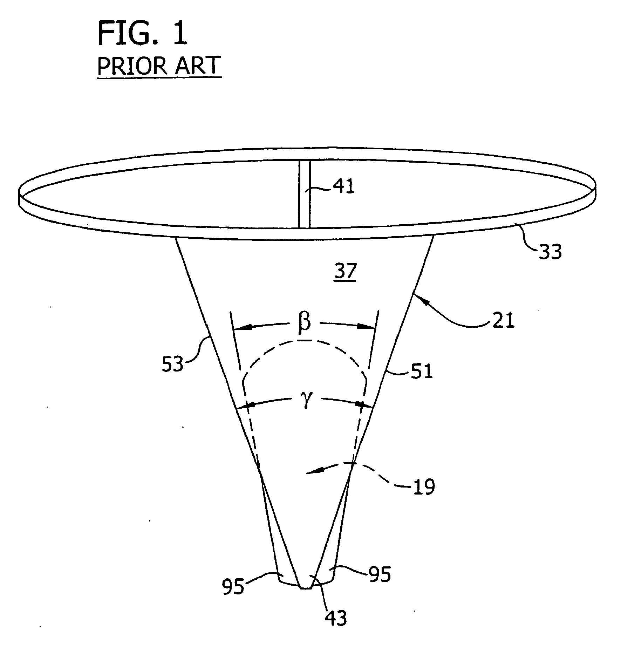

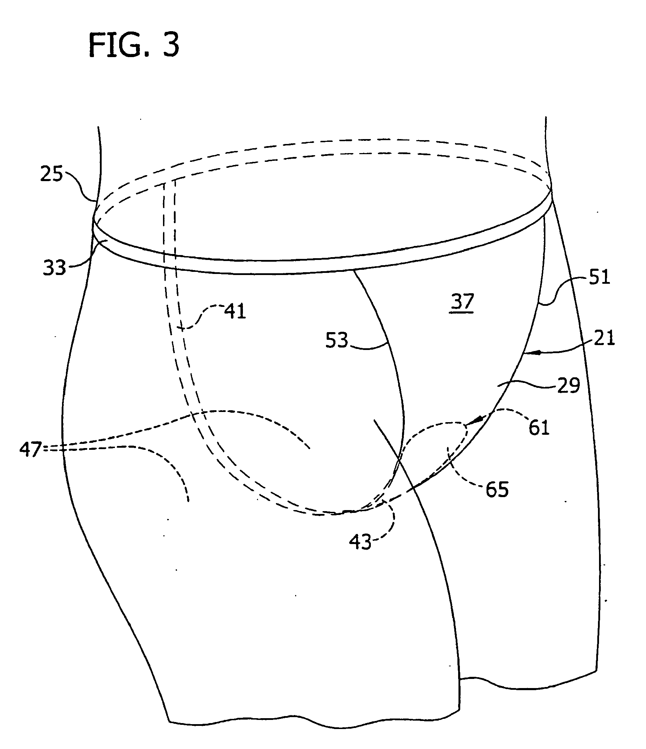

Referring first to Fig. 1, there is illustrated a conventional liner, generally designated 19, designed for use in a thong underwear garment, generally designated 21. Thong underwear 21 fits about a wearer's waist 25 and crotch 29 (Fig. 3), much like traditionally shaped underwear. Thong underwear 21 includes a waistband 33 for encircling the waist 25 of the wearer and a crotch portion 37 extending downward from the waistband for positioning over the crotch 29 of the wearer. A web 41 extends rearward from a lower end 43 of the crotch portion 37 and passes between the buttocks 47 of the wearer for attachment to the waistband 33. It is contemplated that the web 41 could be made in any number of configurations without departing from the scope of the present invention, including, for example, a strap, a strip, a band or a string of material.

The crotch portion 37 of the thong underwear 21 further includes a left garment edge 51 and a right garment edge 53 (left and right from the perspective of the wearer and as viewed in Figs. 4 and 5, for example) converging downward from the waistband 33 toward the lower end 43 of the crotch portion. The crotch portion 37 is similar to traditionally shaped underwear, except that the angle of inclusion γ between the left garment edge 51 and the right garment edge 53 may be larger, such that the lower end 43 of the crotch portion is narrower (Figs. 1, 4 and 5). For example, the angle of inclusion of a crotch portion of traditionally shaped underwear may be between about 12° and about 20° because the crotch portion need not become as narrow between the legs and buttocks of the wearer. In contrast, the angle of inclusion γ of the crotch portion 37 of thong underwear 21 may be between about 22° and about 37°, thereby allowing the lower end of the crotch portion to become narrower than traditionally shaped underwear.

Referring now to Figs. 2-5, a liner, generally designated 61, may be used in combination with the thong underwear 21 described above. Figure 5 illustrates a liner in accordance with the invention. Figures 2 to 4 illustrate a liner not in accordance with the invention, but have been retained to facilitate understanding of certain features of the invention. The liner 61 is adapted for insertion into the crotch portion 37 of the thong underwear 21 and is preferably formed from an absorbent material that absorbs bodily fluids and protects the thong underwear and other adjacent garments from such fluids. Such fluids may be associated with menstruation, ovulation, urine loss or any other process that creates bodily fluid. For example, the liner 61 may be a menstrual pad, a pantyliner or other similar product. The liner 61 is generally flat and includes a garment-facing surface 65 adapted for flatwise contact with the crotch portion 37 of the thong underwear 21 (Figs. 2 and 3). The garment-facing surface 65 has adhesive areas 67 for adhering and securing the liner 61 to the crotch portion 37 of the underwear 21 (Fig. 2). Such adhesive areas 67 are well known in the art and will not be described in detail here. A body-facing surface 71 opposite the garment-facing surface 65 is adapted for flatwise contact with the crotch 29 of the wearer (Figs. 4 and 5).

The liner 61 has a left liner edge 75 and a right liner edge 77 (left and right from the perspective of the wearer and as viewed in Figs. 4 and 5, for example) converging downward toward an apex 79 of the liner. The liner 61 has a size and configuration such that upon insertion of the liner into the crotch portion 37 of the thong underwear 21, the liner apex 79 contacts the crotch portion at a location adjacent the lower end 43 of the crotch portion (Fig. 5). Contrary to conventional liners 19, however, the left liner edge 75 does not extend laterally out beyond the left garment edge 51. Similarly, the right liner edge 77 does not extend laterally out beyond the right garment edge 53. The liner apex 79 also does not overlap with the web 41, but rather is either coterminous with the web as shown in Fig. 5 or spaced sightly forward of the web as shown in Fig. 4. This combination of fit characteristics ensures that the liner does not extend laterally beyond the edges 51,53 of the thong underwear or onto the web 41, thereby minimizing irritation of the wearer.

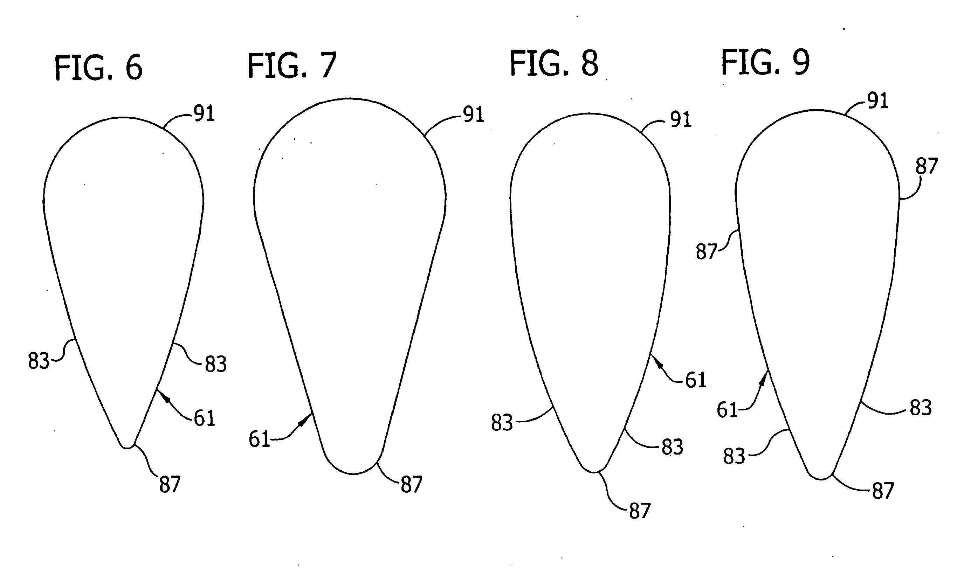

In the configurations in accordance with the invention shown in Figs. 5, 6, 8 and 9, both left and right liner edges 75,77 include a substantially convex portion 83. Such convex portions may be used in conjunction with one or more straight portions 87 (e.g. Fig. 9) and/or one or more arcuate portions to form a left or right liner edge 75, 77. The convex portions 83 may be formed by any suitable curves, but preferably each portion comprises a circular arc. For instance, such an arc may have a radius of at least 100 millimeters (3.9 inches), and preferably includes a radius of at least 150 millimeters (5.9 inches). In other arrangements not in accordance with the invention (e.g.; Fig. 7), the left and right liner edges 75,77 may be substantially straight. In each of the disclosed embodiments, however, the left and right liner edges 75,77 are substantially without corners.

Although the apex 79 of the liner 61 may be pointed or flat, the apex of the liner is preferably arcuate (Figs. 4-9). In addition, the liner apex 79 and the left and right liner edges 75,77 preferably meet to define a substantially smooth liner lower edge 87. This adds to the overall appearance of the liner 61 and avoids sharp corners on the edge of the liner which might otherwise irritate the wearer. More preferably, the liner apex 79 comprises a circular arc.

The liner further comprises an upper liner edge 91 extending between an upper end of the left liner edge 75 and an upper end of the right liner edge 77 (Figs. 4-9). The upper liner edge 91 is preferably arcuate, and more preferably comprises a circular arc. As with the apex 79, the upper liner edge 91 and left and right liner edges 75,77 meet to define a substantially smooth transition to add to the appearance, fit and feel of the liner 61.

In order for the liner 61 to fit properly within the thong underwear 21, the shape of the liner must closely match the shape of the crotch portion 37. Referring now to Figs. 4 and 5, a curvature ratio is hereby defined as the ratio between a radius Rᵤ of the upper liner edge 91 and a radius Rₐ of the liner apex 79. This ratio indicates the degree of taper of the liner 61 along its length L. For instance, a larger ratio indicates a liner 61 having a significant taper from its upper edge 91 to the apex 79. For example, the liners 61 of Figs. 6, 7, 8 and 9 have curvature ratios of 5.3, 3.1, 5.3 and 5.4, respectively. Generally, it is preferred that liners 61 of the present invention have curvature ratios between about 3 and about 7, more preferably between about 4 and about 6 and even more preferably between about 5.0 and about 5.5. Liners 61 having curvature ratios in such ranges are capable of providing the benefits noted above. Conversely, a smaller ratio (e.g., approaching unity) indicates a liner having a less significant taper. Liners designed for traditionally shaped underwear typically have ratios at or approaching unity and are not suitable for thong underwear 21, as discussed above.

Another measure of the liner's ability to match the shape of the crotch portion 37 without extending laterally out beyond the left and right garment edges 51,53 is an angle of inclusion β of the liner 61. The angle of inclusion of previous liner designs for thong underwear tended to be smaller than optimal. For example, the liner 19 of Fig. 1 has an angle of inclusion β of about 19°. Such an angle of inclusion β causes lower side portions 95 of the liner 19 to extend beyond the lateral edges 51,53 of the crotch portion 37. As discussed above, such a design may cause wearer irritation. The angle of inclusion β for liners 61 of the present invention, however, are larger and therefore provide a better fit with thong underwear 21.

Measuring an angle of inclusion β for a liner 61 having substantially straight left and right liner edges 75,77 is relatively simple, as it is simply the included angle between the straight edges. For example, the angle of inclusion β of the liner 61 depicted in Fig. 7 is 32.4°. For a liner 61 having non-linear edges 75,77, such as those depicted in Figs. 6, 8 and 9, the angle of inclusion β must be defined differently, because the angularity of the edges changes along their length. As used herein, the term "angle of inclusion" β for non-linear edges 75,77 is measured using lines drawn between defined points on the liner 61 (Fig. 5). A first set of points, P_{w}, corresponds to the widest portion of the liner 61. A second set of points, Pₐ, corresponds to an intersection of the liner edge 75,77 with a line perpendicular to a longitudinal axis A of the liner 61 and passing through the center C of the circular apex 79. By measuring the angle between lines P_{w}-Pₐ, the angle of inclusion β of a liner 61 with non-linear edges 75,77 is readily defined. For example, the liners 61 of Figs. 6, 8 and 9 have angles of inclusion β of 34.6°, 28.4° and 28.7°, respectively. Generally speaking, an angle of inclusion β for a liner 61 of the present invention is between about 27° and about 35° and even more preferably between about 28° and about 33°.

Liner shape may also be defined with an aspect ratio comparing a maximum length L of the liner 61 and a maximum width W of the liner. For example, the liners 61 of Figs. 6, 7, 8 and 9 include aspect ratios of 2.0, 1.9, 2.1 and 2.2, respectively. For the present invention, an appropriate aspect ratio is preferably between about 1.5 and about 2.5, even more preferably between about 2.0 and about 2.2 and still more preferably between about 1.8 and about 1.9. Each of the foregoing measures provides some indication of the ability of a particular liner 61 to fit concealed within thong underwear 21.

The desired use of the liner 61 largely determines its required thickness. In general, a liner 61 of the present invention may have a thickness of between about 0.5 millimeter (0.02 inch) and about 20 millimeters (0.8 inch), more preferably a thickness of between about 0.5 millimeter (0.02 inch) and about 12 millimeters (0.47 inch), even more preferably a thickness of between about 0.5 millimeter (0.02 inch) and about 2.0 millimeters (0.079 inch) and still more preferably a thickness of between about 0.75 millimeter (0.030 inch) and about 1.5 millimeter (0.059 inch). For a pantyliner, which needs less absorptive capacity, the thickness of the liner 61 is preferably between about 0.5 millimeter (0.02 inch) and about 2.0 millimeters (0.079 inch). For a menstrual pad, which needs more absorptive capacity, the thickness of the liner 61 is preferably between about 2.0 millimeters (0.079 inch) and about 20 millimeters (0.8 inch).

The liner 61 of the present invention is preferably formed from flexible, soft material or materials so that the liner readily conforms to the contour of the thong underwear 21 and the crotch 29 of the wearer. The body-facing surface 71 is preferably made from a cotton material, or a material exhibiting cottony characteristics, such as a nonwoven material. The liner 61 also preferably comprises an absorbent material capable of absorbing bodily fluids. For instance, both an airlaid material with a mass per area of between about 30 grams per square meter (gsm) (0.9 ounces per square yard (osy)) and about 100 gsm (2.9 osy) or a Thru-Air Bonded Carded Web (TABCW) material are appropriate choices. For additional detail regarding the construction of liners, reference may be made to U.S. Patent Nos. 4,079,739, entitled DIE-CUT CONTOURED CATAMENIAL NAPKIN OF MULTI-LAYERED CONSTRUCTION, by Whitehead, assigned to Kimberly-Clark Worldwide, Inc., 4,758,239, entitled BREATHABLE BARRIER, by Yeo, et al., assigned to Kimberly-Clark Worldwide, Inc., 6,172,276, entitled STABILIZED ABSORBENT MATERIAL FOR IMPROVED DISTRIBUTION PERFORMANCE WITH VISCO-ELASTIC FLUIDS, by Hetzler, et al., and 5,429,630, entitled ABSORBENT ARTICLE AND A METHOD OF REMOVING SAID ARTICLE FROM AN UNDERGARMENT, by Beal, et al., assigned to Kimberly-Clark Worldwide, Inc. It will be understood, however, that the principles of the present invention can be practiced with different liner constructions.

Although the liner 61 of the present invention is specifically designed to fit within thong underwear 21, it will be understood by those skilled in the art that such a liner could readily be inserted into and used with traditionally shaped underwear without departing from the scope of the present invention.

The liners 61 depicted in the figures are exemplary only. Other liners exhibiting the characteristics and features of the present invention are also contemplated as within the scope of the present invention.

In view of the above, it will be seen that the several objects of the invention are achieved and other advantageous results attained.

When introducing elements of the present invention or the preferred embodiment(s) thereof, the articles "a", "an", "the" and "said" are intended to mean that there are one or more of the elements. The terms "comprising", "including" and "having" are intended to be inclusive and mean that there may be additional elements other than the listed elements.

As various changes could be made in the above without departing from the scope of the invention, it is intended that all matter contained in the above description and shown in the accompanying drawings shall be interpreted as illustrative and not in a limiting sense.

## Claims

1. A liner (61) adapted for insertion into a crotch portion (37) of a garment (21) for absorbing bodily fluids and protecting said garment from such fluids, said liner (61) being generally flat and including a garment-facing surface adapted for flatwise contact with a crotch portion of the garment and a body-facing surface adapted for flatwise contact with a crotch of a wearer, said liner further including a left liner edge (75) and a right liner edge (77) converging downward toward an apex (79) of said liner, said liner further comprising an arcuate upper liner edge (91) extending between an upper end of the left liner edge (75) and an upper end of the right liner edge (79),
**characterised in that** an angle of inclusion (β) between said left liner edge (75) and said right liner edge (77) is between about 27° and about 35°; and
wherein said left and right liner edges (75, 77) both include a substantially convex portion (83).

2. A liner as set forth in claim 1 wherein said angle of inclusion (β) is between about 28° and about 33°.

3. A liner as set forth in claim 1 or 2 wherein said liner apex (79) comprises a circular arc.

4. A liner as set forth in claim 1 wherein said upper liner edge (91) comprises a circular arc.

5. A liner as set forth in claims 4 wherein a curvature ratio between a radius of said upper liner edge (91) and a radius of said liner apex (79) is between about 3 and about 7.

6. A liner as set forth in claim 5 wherein said curvature ratio is between about 4 and about 6.

7. A liner as set forth in claim 6 wherein said curvature ratio is between about 5.0 and about 5.5.

8. A liner as set forth in any preceding claim wherein said substantially convex portions (83) comprise circular arcs, each having a radius of at least 100 millimeters (3.9 inches).

9. A liner as set forth in any of claim 1 to 7 wherein said substantially convex portions (83) comprise circular arcs, each having a radius of at least 150 millimeters (5.9 inches).

10. A liner as set forth in any preceding claim wherein said left and right liner edges (75, 77) are substantially without corners.

11. A liner as set forth in any preceding claim wherein said liner apex (79) and said left and right liner edges meet to define a substantially smooth liner lower edge.

12. A liner (61) as set forth in any preceding claim wherein said liner is a pantyliner.

13. A liner (61) as set forth in any of claims 1 to 11 wherein said liner is a menstrual pad.

14. A liner as set forth in any preceding claim wherein said upper liner edge (91) and said left and right liner edges (75, 77) meet to define a substantially smooth transition between said liner edges.

15. A liner (61) as set forth in any of claims 1 to 11 wherein said liner has a thickness of between about 0.5 millimeter (0.02 inch) and about 20 millimeters (0.8 inch).

16. A liner (61) as set forth in claim 15 wherein said liner has a thickness of between about 0.5 millimeter (0.02 inch) and about 12 millimeters (0.47 inch).

17. A liner (61) as set forth in claim 16 wherein said liner has a thickness of between about 0.5 millimeter (0.02 inch) and about 2.0 millimeters (0.079 inch).

18. A liner (61) as set forth in claim 17 wherein said liner has a thickness of between about 0.75 millimeter (0.030 inch) and about 1.5 millimeter (0.059 inch).

19. The liner of any preceding claim wherein said garment-facing surface (65) has adhesive areas (67) for adhering and securing the liner (61) to the crotch portion (37) of the garment.

20. The liner of any preceding claim in combination with a garment (21) for fitting about a wearer's waist (25) and crotch (29), the garment including a waistband (33) for encircling the waist (25) of said wearer, a crotch portion (37) extending downward from said waistband for positioning over the crotch (29) of the wearer, said crotch portion (37) including a left garment edge and a right garment edge converging downward from the waistband toward a lower end of said crotch portion, and a web (41) extending rearward from said lower end (43) of said crotch portion (37) for passing between the buttocks (47) of the wearer and attaching to the waistband,
said liner having a size and configuration such that upon insertion of the liner into said crotch portion of the garment, the liner apex (79) contacts the crotch portion at a location adjacent the lower end of the crotch portion, said left liner edge (75) does not extend laterally out beyond the left garment edge, said right liner edge (77) does not extend laterally out beyond the right garment edge, and said liner apex (79) is not overlapping with said web.

21. A combination as set forth in claim 20 wherein an angle of inclusion (γ) between said left garment edge and said right garment edge is between about 22° and about 37°.

## Patentansprüche

1. Einlage (61) zum Einsetzen in einen Schrittabschnitt (37) eines Kleidungsstückes (21), um Körperflüssigkeiten zu absorbieren und das Kleidungsstück vor diesen Flüssigkeiten zu schützen, wobei die Einlage (61) im Allgemeinen flach ist und eine dem Kleidungsstück zugewandte Oberfläche, die für den flachseitigen Kontakt mit einem Schrittabschnitt des Kleidungsstückes eingerichtet ist, und eine dem Körper zugewandte Oberfläche aufweist, die für den flachseitigen Kontakt mit dem Schritt eines Trägers eingerichtet ist, wobei die Einlage ferner einen linken Einlagenrand (75) und einen rechten Einlagenrand (77) aufweist, die zu einem abwärts gelegenen Scheitelpunkt (79) der Einlage zusammenlaufen, wobei die Einlage ferner einen bogenförmigen oberen Einlagenrand (91) umfasst, der sich zwischen einem oberen Ende des linken Einlagenrandes (75) und einem oberen Ende des rechten Einlagenrandes (79) erstreckt,
**dadurch gekennzeichnet, dass** ein Einschlusswinkel (β) zwischen dem linken Einlagenrand (75) und dem rechten Einlagenrand (77) zwischen etwa 27° und etwa 35° groß ist, und
wobei sowohl der linke als auch der rechte Einlagenrand (75, 77) einen im Wesentlichen konvexen Abschnitt (83) umfassen.

2. Einlage nach Anspruch 1, wobei der Einschlusswinkel (β) zwischen etwa 28° und etwa 33° groß ist.

3. Einlage nach Anspruch 1 oder 2, wobei der Einlagenscheitelpunkt (79) einen Kreisbogen umfasst.

4. Einlage nach Anspruch 1, wobei der obere Einlagenrand (91) einen Kreisbogen umfasst.

5. Einlage nach Anspruch 4, wobei ein Krümmungsverhältnis zwischen einem Radius des oberen Einlagenrandes (91) und einem Radius des Einlagenscheitelpunktes (79) zwischen etwa 3 und etwa 7 liegt.

6. Einlage nach Anspruch 5, wobei das Krümmungsverhältnis zwischen etwa 4 und etwa 6 liegt.

7. Einlage nach Anspruch 6, wobei das Krümmungsverhältnis zwischen etwa 5,0 und etwa 5,5 liegt.

8. Einlage nach einem der vorhergehenden Ansprüche, wobei die im Wesentlichen konvexen Abschnitte (83) Kreisbögen umfassen, die jeweils einen Radius von mindestens 100 Millimetern (3,9 Inch) aufweisen.

9. Einlage nach einem der Ansprüche 1 bis 7, wobei die im Wesentlichen konvexen Abschnitte (83) Kreisbögen umfassen, die jeweils einen Radius von mindestens 150 Millimetern (5,9 Inch) aufweisen.

10. Einlage nach einem der vorhergehenden Ansprüche, wobei der linke und der rechte Einlagenrand (75, 77) im Wesentlichen ohne Ecken sind.

11. Einlage nach einem der vorhergehenden Ansprüche, wobei sich der Einlagenscheitelpunkt (79) und der linke und der rechte Einlagenrand treffen, um einen im Wesentlichen glatten unteren Rand der Einlage zu definieren.

12. Einlage (61) nach einem der vorhergehenden Ansprüche, wobei die Einlage eine Slipeinlage ist.

13. Einlage (61) nach einem der Ansprüche 1 bis 11, wobei die Einlage eine Menstruationsbinde ist.

14. Einlage nach einem der vorhergehenden Ansprüche, wobei sich der obere Einlagenrand (91) und der linke und der rechte Einlagenrand (75, 77) treffen, um einen im Wesentlichen glatten Übergang zwischen den Einlagenrändern zu definieren.

15. Einlage (61) nach einem der Ansprüche 1 bis 11, wobei die Einlage eine Dicke zwischen etwa 0,5 Millimeter (0,02 Inch) und etwa 20 Millimeter (0,8 Inch) aufweist.

16. Einlage (61) nach Anspruch 15, wobei die Einlage eine Dicke zwischen etwa 0,5 Millimeter (0,02 Inch) und etwa 12 Millimeter (0,47 Inch) aufweist.

17. Einlage (61) nach Anspruch 16, wobei die Einlage eine Dicke zwischen etwa 0,5 Millimeter (0,02 Inch) und etwa 2,0 Millimeter (0,079 Inch) aufweist.

18. Einlage (61) nach Anspruch 17, wobei die Einlage eine Dicke zwischen etwa 0,75 Millimeter (0,030 Inch) und etwa 1,5 Millimeter (0,059 Inch) aufweist.

19. Einlage nach einem der vorhergehenden Ansprüche, wobei die dem Kleidungsstück zugewandte Oberfläche (65) Haftbereiche (67) zum Anheften und Befestigen der Einlage (61) am Schrittabschnitt (37) des Kleidungsstückes aufweist.

20. Einlage nach einem der vorhergehenden Ansprüche in Kombination mit einem Kleidungsstück (21) zum Überziehen über die Taille (25) und den Schritt (29) eines Trägers, wobei das Kleidungsstück ein Taillenband (33) aufweist, das die Taille (25) des Trägers umgibt, einen Schrittabschnitt (37), der sich vom Taillenband abwärts erstreckt, um über dem Schritt (29) des Trägers positioniert zu sein, wobei der Schrittabschnitt (37) einen linken Kleidungsstückrand und einen rechten Kleidungsstückrand, die abwärts vom Taillenband zu einem unteren Ende des Schrittabschnittes zusammenlaufen, und einen Steg (41) aufweist, der sich vom unteren Ende (43) des Schrittabschnittes (37) nach hinten erstreckt, um zwischen den Gesäßbacken (47) des Trägers zu verlaufen und am Taillenband befestigt zu sein,
wobei die Einlage eine derartige Größe und Gestaltung aufweist, dass nach dem Einsetzen der Einlage in den Schrittabschnitt des Kleidungsstückes der Einlagenscheitelpunkt (79) den Schrittabschnitt an einer Stelle berührt, die an das untere Ende des Schrittabschnittes angrenzt, wobei sich der linke Einlagenrand (75) seitlich nicht über den linken Kleidungsstückrand hinaus erstreckt, wobei sich der rechte Einlagenrand (77) seitlich nicht über den rechten Kleidungsstückrand hinaus erstreckt und der Einlagenscheitelpunkt (79) den Steg nicht überlappt.

21. Kombination nach Anspruch 20, wobei ein Einschlusswinkel (γ) zwischen dem linken Kleidungsstückrand und dem rechten Kleidungsstückrand zwischen etwa 22° und etwa 37° groß ist.

## Revendications

1. Revêtement (61) destiné à être inséré dans une partie d'entrejambes (37) d'un sous-vêtement (21) pour absorber les fluides corporels et protéger ledit sous-vêtement de ces fluides,
ledit revêtement (61) étant globalement plat et comprenant face au sous-vêtement une surface conçue pour être en contact de surface avec une partie d'entrejambes du sous-vêtement et face au corps une surface conçue pour être en contact de surface avec l'entrejambes de la personne portant le sous-vêtement,
ledit revêtement comprenant de plus un bord gauche (75) de revêtement et un bord droit (77) de revêtement convergeant vers le bas vers un sommet (79) dudit revêtement,
ledit revêtement comprenant de plus un bord supérieur incurvé (91) de revêtement s'étendant entre une extrémité supérieure du bord gauche (75) du revêtement et une extrémité supérieure du bord droit (79) du revêtement,
**caractérisé en ce que**
l'angle (β) inclus entre ledit bord gauche (75) du revêtement et ledit bord droit (77) du revêtement est compris entre environ 27° et environ 35° et
**en ce que** les bords gauche et droit (75, 77) du revêtement comprennent chacun une partie (83) essentiellement convexe.

2. Revêtement selon la revendication 1, dans lequel ledit angle inclus (β) est compris entre environ 28° et environ 33°.

3. Revêtement selon les revendications 1 ou 2, dans lequel ledit sommet (79) du revêtement comprend un arc de cercle.

4. Revêtement selon la revendication 1, dans lequel ledit bord supérieur (91) du revêtement comprend un arc de cercle.

5. Revêtement selon la revendication 4, dans lequel le rapport de courbure entre le rayon dudit bord supérieur (91) du revêtement et le rayon dudit sommet (79) du revêtement est compris entre environ 3 et environ 7.

6. Revêtement selon la revendication 5, dans lequel ledit rapport de courbure est compris entre environ 4 et environ 6.

7. Revêtement selon la revendication 6, dans lequel ledit rapport de courbure est compris entre environ 5,0 et environ 5,5.

8. Revêtement selon l'une quelconque des revendications précédentes, dans lequel lesdites parties (83) essentiellement convexes comprennent des arcs de cercle, chacun d'un rayon d'au moins 100 millimètres (3,9 pouces).

9. Revêtement selon l'une quelconque des revendications 1 à 7, dans lequel lesdites parties (83) essentiellement convexes comprennent des arcs de cercle, chacun d'un rayon d'au moins 150 millimètres (5,9 pouces).

10. Revêtement selon l'une quelconque des revendications précédentes, dans lequel lesdits bords gauche et droit (75, 77) du revêtement sont essentiellement sans coin.

11. Revêtement selon l'une quelconque des revendications précédentes, dans lequel ledit sommet (79) du revêtement et lesdits bords gauche et droit du revêtement se rencontrent pour définir des bords inférieurs de revêtement essentiellement lisses.

12. Revêtement (61) selon l'une quelconque des revendications précédentes, dans lequel ledit revêtement est un protège-slip.

13. Revêtement (61) selon l'une quelconque des revendications 1 à 11, dans lequel ledit revêtement est une serviette hygiénique.

14. Revêtement selon l'une quelconque des revendications précédentes, dans lequel ledit bord supérieur (91) du revêtement et lesdits bords gauche et droit (75, 77) du revêtement se rencontrent pour définir une transition essentiellement lisse entre lesdits bords de revêtement.

15. Revêtement (61) selon l'une quelconque des revendications 1 à 11, dans lequel ledit revêtement présente une épaisseur comprise entre environ 0,5 millimètre (0,02 pouce) et environ 20 millimètres (0,8 pouce).

16. Revêtement (61) selon la revendication 15, dans lequel ledit revêtement présente une épaisseur comprise entre environ 0,5 millimètre (0,02 pouce) et environ 12 millimètres (0,47 pouce).

17. Revêtement (61) selon la revendication 16, dans lequel ledit revêtement présente une épaisseur comprise entre environ 0,5 millimètre (0,02 pouce) et environ 2,0 millimètres (0,079 pouce).

18. Revêtement (61) selon la revendication 17, dans lequel ledit revêtement présente une épaisseur comprise entre environ 0,75 millimètre (0,030 pouce) et environ 1,5 millimètre (0,059 pouce).

19. Revêtement selon l'une quelconque des revendications précédentes, dans lequel ladite surface (65) faisant face au sous-vêtement présente des surfaces adhésives (67) de manière à faire adhérer et à fixer le revêtement (61) à la partie d'entrejambes (37) du sous-vêtement.

20. Revêtement selon l'une quelconque des revendications précédentes, en combinaison avec un sous-vêtement (21) destiné à être placé autour de la taille (25) et de l'entrejambes (29) d'une personne, le sous-vêtement comprenant une bande de taille (33) qui encercle la taille (25) dudit porteur, une partie d'entrejambes (37) s'étendant vers le bas à partir de ladite bande de taille permettant un positionnement sur l'entrejambes (29) du porteur, ladite partie d'entrejambes (37) comprenant un bord gauche de revêtement et un bord droit de revêtement convergeant vers le bas à partir de la bande de taille vers une extrémité inférieure de ladite partie d'entrejambes et une nappe (41) qui s'étend vers l'arrière à partir de ladite extrémité inférieure (43) de ladite partie d'entrejambes (37) passant entre les fesses (47) du porteur et se reliant à la bande de taille,
ledit revêtement présentant une taille et une configuration telles que lors du placement du revêtement dans ladite partie d'entrejambes du sous-vêtement, le sommet (79) du revêtement entre en contact avec la partie d'entrejambes en un emplacement situé juste à côté de l'extrémité inférieure de la partie d'entrejambes, ledit bord gauche (75) du revêtement ne s'étendant pas latéralement au-delà du bord gauche de sous-vêtement, ledit bord droit (77) du revêtement ne s'étendant pas latéralement au-delà du bord droit de sous-vêtement et ledit sommet (79) du revêtement ne recouvrant pas ladite nappe.

21. Combinaison selon la revendication 20 dans laquelle un angle (γ) inclus entre ledit bord gauche du sous-vêtement et ledit bord droit du sous-vêtement est compris entre 22° et environ 37°.
